# EUROPÄISCHE PATENTSCHRIFT

(11) **EP 1 962 781 B1**
(45) Veröffentlichungstag und Bekanntmachung des Hinweises auf die Patenterteilung: **14.05.2014**
(21) Anmeldenummer: 06830556.4
(22) Anmeldetag: 12.12.2006
(51) Int. Cl.: A61K 8/35, A61K 8/37, A61K 8/49, A61Q 17/04, A61K 8/02, A61K 8/34, A61K 8/40

(54) **Transparentes Sonnenschutzmittel**
Transparent sunscreen agents
Produit de protection solaire transparent

(30) Priorität: 13.12.2005 DE 102005059742
(43) Veröffentlichungstag der Anmeldung: 03.09.2008
(73) Patentinhaber: Beiersdorf AG, 20253 Hamburg (DE)
(72) Erfinder: LERG, Heike, 22303 Hamburg (DE); MUNDT, Claudia, 28207 Bremen (DE); SUGÁR, Martin, 20257 Hamburg (DE); SKUBSCH, Kerstin, 25421 Pinneberg (DE); TESCH, Mirko, 22303 Hamburg (DE); WINDISCH, Björna, 22607 Hamburg (DE)
(86) Internationale Anmeldenummer: PCT/EP2006/069608
(87) Internationale Veröffentlichungsnummer: WO 2007/068699

(56) Entgegenhaltungen:
- EP-A- 0 812 586
- EP-A- 1 579 847
- EP-A- 1 586 306
- WO-A-03/053393
- WO-A-2005/107693
- DE-A1- 10 327 468
- DE-A1-102004 009 154

## Beschreibung

Die vorliegende Erfindung betrifft ein transparentes Sonnenschutzmittel.

Der Trend weg von der vornehmen Blasse hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schadigende Wirkung auf die Haut. Neben der akuten Schädigung (Sonnenbrand) treten Langzeitschaden wie ein erhöhtes Risiko an Hautkrebs zu erkranken bei übermäßiger Bestrahlung mit Licht aus dem UVB-Bereich (Wellenlange: 280-320 nm) auf. Die ubermäßige Einwirkung der UVB- und UVA-Strahlung (Wellenlange 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können Diese UVA- und UVB-Filter sind in den meisten Industrieländern in Form von Positivlisten wie der Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. So ist es bisher nicht möglich gewesen, transparente Sonnenschutzmittel (beispielsweise Gele) mit hohem UV-A Schutz herzustellen. Transparente kosmetische Zubereitungen werden in der Regel auf Wasserbasis formuliert. Auch sind dem Fachmann alkoholische (ethanolische) oder wässrig-alkoholische Zubereitungen bekannt. Ethanolische Zubereitungen haben den großen Vorteil, dass sie auf der Haut besonders schnell "einsatzfertig" sind und einen erfrischend-kühlen sensorischen Eindruck hinterlassen. Beides ist auf die hohe Flüchtigkeit (schnelle Verdunstung) des Alkohols zurückzufuhren. Nachteilig am Stande der Technik ist jedoch der Umstand, dass sich allenfalls geringe Mengen an UV-A-Lichtschutzfiltersubstanzen in alkoholische Zubereitungen stabil einarbeiten lassen. Bei höheren Konzentrationen fallen diese Filter leicht aus der Zubereitung wieder aus. Dieser Effekt tritt insbesondere bei einer kühlen Lagerung (z.B. beim Transport oder in unbeheizten Lagerräumen) auf. Bei der Anwendung derartiger Zubereitungen des Standes der Technik auf der Haut kommt es darüber hinaus nach der Verdunstung des Alkohols zu einer ungleichmäßigen Ablagerung (Aggregation) der Filtersubstanzen auf der Haut, welche sich in der Form sichtbarer weißer Flecken und Ringe auf der Haut, dem so genannten "weißeln", äußert. Um diese Nachteile zu vermeiden hat man bisher auf den Einsatz höherer Konzentrationen an UV-A-Lichtschutzfiltern in alkoholischen Zubereitungen verzichtet, so dass bis heute keine alkoholischen Zubereitungen angeboten werden, die eine ausgewogene UV-A-Balance (nach DIN 67502) aufweisen und/oder den australischen Standard AS 2604:93, d.h. eine UV-A-Strahlen-Absorption von mindestens 90 %, erfüllen.

Es war daher die Aufgabe der vorliegenden Erfindung eine kosmetische Zubereitung welche die menschliche Haut vor der UV-Strahlung schützt (im Rahmen dieser Offenbarung als "Sonnenschutzmittel" bezeichnet) auf Ethanolbasis zu entwickeln, die
■ einen hohen Schutz vor UV-A-Strahlung bietet,
■ den australischen Standard AS 2604:93 für den Schutz vor UV-A-Strahlung erfüllt,
■ eine hohe UV-A-Balance (nach DIN 67502) d.h. eine UV-A-Balance von mindestens 25 aufweist,
■ lagerstabil ist,
■ kälteunempfindlich ist (d.h. eine Lagerung bei -10°C über 1 Monat übersteht, ohne dass es zu Ausfällungen aus der Zubereitung kommt),
■ transportstabil ist,
■ sich unter der Bestrahlung von Tageslicht bei Raumtemperatur (20 °C) über einen längeren Zeitraum (4 Wochen) nicht verfärbt (in diesem Sinne photostabil ist),
■ sich unter der Bestrahlung von Tageslicht in einem klaren Glasgefäß bei Raumtemperatur (20 °C) über einen Zeitraum von 4 Wochen nicht verfärbt (in diesem Sinne farbstabil ist),
■ unter der Bestrahlung mit Tageslicht bei Raumtemperatur über einen längeren Zeitraum (4 Wochen) duftstabil bleibt (d.h. das sich der Geruch der Zubereitung nicht ändert),
■ bei der Anwendung auf der Haut nicht "weißelt".

Überraschend gelöst wird die Aufgabe durch ein kosmetisches Sonnenschutzmittel enthaltend
a) von 20 bis 80 Gewichts-% Ethanol,
b) von 15 bis 45 Gewichts-% bei 20 °C flüssige Ölkomponenten,
c) von 1,0 bis 12 Gewichts-% UV-A-Lichtschutzfilter aus der Gruppe der Verbindungen 2-(4'-Diethylamino-2'-hydoxybenzoyl)-benzoesäurehexylester und 4-(tert.-Butyl)-4'-methoxydibenzoylmethan,
wobei sich die Konzentrationsangaben auf das Gesamtgewicht der Zubereitung beziehen, dadurch gekennzeichnet, dass die bei 20°C flüssigen Ölkomponenten gewählt werden aus der Gruppe der Verbindungen C₁₂₋₁₅-Alkylbenzoat, Octyldodekanol, Phenethylbenzoat, Cocoglycerid, 2-Ethylhexyl-2-cyano-3,3-diphenylacrylat, Homomenthylsalicylat, Ethylhexylsalicylat, 4-Methoxyzimtsäureisoamylester, Dibutyladipat, 4-Methoxyzimtsäure(2-ethylhexyl)ester, Cyclomethicon, Dimethicon, sowie dadurch gekennzeichnet, dass der Wassergehalt der Zubereitung höchstens 4,5 Gewichts-%, bezogen auf das Gesamtgewicht der Zubereitung, beträgt..

Zwar kennt der Fachmann die EP 812586, US 2002/172646, US 5204090, WO 95/25544, US 5888520, WO 98/43673, WO 2004/052353, WO 2001/041727, EP 412705, US 4671955, WO 2003/086342, WO 2001/043722, WO 00/047628, WO 2005/069822 und die DE 103274685, doch konnten diese Schriften nicht den Weg zur vorliegenden Erfindung weisen. Darüber hinaus kannte der Fachmann die EP 1586306, EP 1579847 und WO 03/053393, die ebenfalls nicht den Weg zur vorliegenden Erfindung weisen konnten.

Erfindungsgemäß bevorzugt enthält die erfindungsgemäße Zubereitung von 50 bis 80 Gewichts-% Ethanol und ganz besonders bevorzugt 60-80 Gewichtes-% Ethanol, jeweils bezogen auf das Gesamtgewicht der Zubereitung.

Erfindungsgemäß ganz besonders bevorzugt enthält die erfindungsgemäße Zubereitung 25 bis 45 Gewicht-% bei 20 °C flüssige Ölkomponenten, jeweils bezogen auf das Gesamtgewicht der Zubereitung.

Erfindungsgemäß bevorzugt enthält die erfindungsgemäße Zubereitung von 2 bis 10 Gewichts-% UV-A-Lichtschutzfilter aus der Gruppe der Verbindungen 2-(4'-Diethylamino-2'-hydoxybenzoyl)-benzoesäurehexylester und 4-(tert.-Butyl)-4'-methoxydibenzoylmethan, bezogen auf das Gesamtgewicht der Zubereitung.

Erfindungsgemäß ist ferner ein transparentes kosmetisches Sonnenschutzmittel mit einem Transmissionswert von kleiner 40%.

Das erfindungsgemäßes Sonnenschutzmittel kann in einem transparenten Verpackungsbehältnis enthalten sein.

Der Einsatz von Butylenglykol DicaprylatlDicaprat, Octocrylen, 4-Methoxyzimtsäure(2-ethylhexyl)ester ist erfindungsgemäß besonders bevorzugt.

Ferner ist es erfindungsgemäß besonders bevorzugt, wenn als UV-A-Lichtschutzfilter 4-(tert.-Butyl)-4'-methoxydibenzoylmethan eingesetzt wird.

Es ist erfindungsgemäß, wenn die erfindungsgemäße Zubereitung eine UV-A-Strahlen Absorption von größer oder gleich 90 %, gemessen nach dem australischen Standard AS 2604:93 aufweist.

Außerdem ist es erfindungsgemäß vorteilhaft, wenn die erfindungsgemäße Zubereitung eine UV-A-Balance von mindestens 30 gemessen nach DIN 67502 aufweist.

Die erfindungsgemäße Zubereitung ist transparent ist. Dabei gilt eine Zubereitung erfindungsgemäß als transparent, wenn es möglich ist bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Anal Schrittgröße 8), die sich unmittelbar hinter der Einmal-Küvette befinden, müssen klar erkennbar und lesbar sein.

Obwohl die erfindungsgemäßen Zubereitung dem Betrachter klar und transparent erscheinen, liegen die mittels Photometer HP 8453 ermittelten Transmissionswerte (gemessen wurde bei 420 nm) in einem Bereich kleiner als 40 % und bevorzugt von kleiner 27% (Als Blindwert wurde 100% Ethanol verwendet, eine "weiße" Emulsion hat den Messwert 0%).

Erfindungsgemäß vorteilhafte Ausführungsformen der vorliegenden Erfindung sind ferner dadurch gekennzeichnet, dass die Zubereitung eine Viskosität von 5 mPas bis 2500 mPas aufweist. Dabei wird die erfindungsgemäße Viskosität wie folgt bestimmt: Eine Viskositätsmessung, wird mit dem Viskotester VT 02 der Firma Haake bei 25°C +/- 1°C, Spindel 1 durchgeführt. Die Ablesung erfolgt nach 30 Sekunden.

Die erfindungsgemäßen Sonnenschutzmittel können erfindungsgemäß vorteilhaft weitere UV-Lichtschutzfiltersubstanzen enthalten.

So sind erfindungsgemäß vorteilhafte Ausführungsformen der vorliegenden Erfindung dadurch gekennzeichnet, dass die Zubereitung einen oder mehrere weitere UV-Lichtschutzfilter gewählt aus der Gruppe der Verbindungen Dioctylbutylamidotriazon; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-'2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl,Triazone); Merocyanine, 2,4,6-Tribiphenyl-4-yl-1,3,5-triazin; Titandioxid; Zinkoxid in einer Konzentration von 0,01 bis 40 Gewichts-%, bezogen auf das Gesamtgewicht der Zubereitung, enthält.

Enthält die Zubereitung Merocyanine; so ist der Einsatz der folgenden Verbindungen erfindungsgemäß bevorzugt:

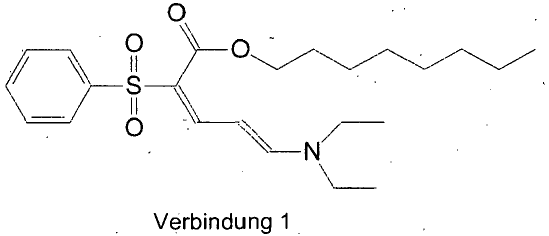

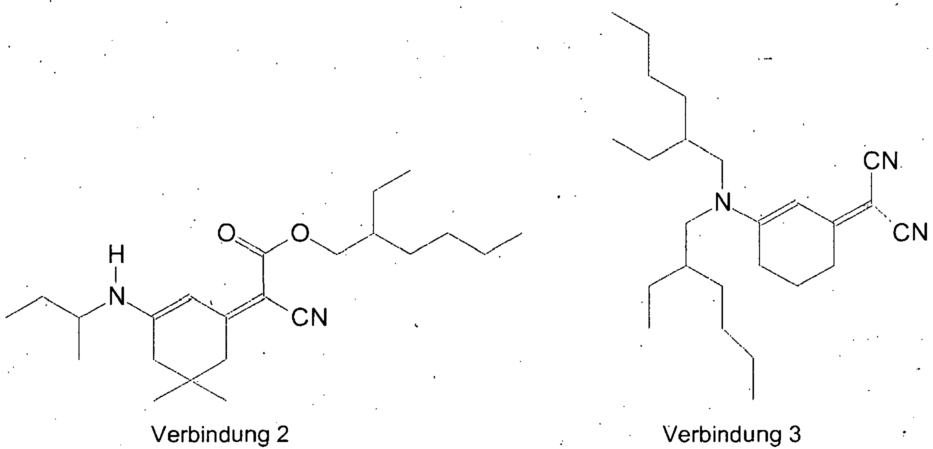

Erfindungsgemäß vorteilhaft enthält die erfindungsgemaße Zubereitung kein 2,4,6-Tris-[anilino-(p-carbo-2'-ethyl-1'-hexyloxy)]-1,3,5-triazin.

Erfindungsgemaß vorteilhafte Ausführungsformen der vorliegenden Erfindung sind dadurch gekennzeichnet, dass die Zubereitungen kein Aminomethylpropanol, Tetrahydroxypropyl ethylendiamin, Diisopropanolamin, Triisopropanolamin, PEG-15 Cocamin oder deren Salze enthalten.

Die erfindungsgemaße Zubereitung kann erfindungsgemaß vorteilhaft in zwei unterschiedlichen Ausführungsformen vorliegen. Wird die Zubereitung als Spray verwendet, so enthält sie erfindungsgemäß vorteilhaft keine Verdicker. Wird die Zubereitung jedoch nicht verspruht, ist es erfindungsgemäß vorteilhaft, ihr ein oder mehrere Verdicker zuzusetzen.

Es ist erfindungsgemäß vorteilhaft, wenn die erfindungsgemäße Zubereitung einen oder mehrere Verdickungsmittel gewahlt aus der Gruppe der Hyaluronsaure, Xanthangummi, Hydroxypropylmethylcellulose, Hydroxypropylcellulose, Ethylcellulose, Acrylat / C10-30 Alkyl Acrylat Crosspolymer (Handelsnamen: Carbopol Ultrez 10 oder Carbopol ETD) besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination, enthält. Weitere erfindungsgemäß vorteilhafte Verdicker sind Permulen TR 1, TR 2, Carbopol 1328, Anstoflex AVC. Erfindungsgemäß besonders bevorzugt ist der Einsatz von Hydroxypropylcellulose als Verdicker.

Vorteilhafte Ausführungsformen, welche Verdicker enthalten, sind dabei dadurch gekennzeichnet, dass die Zubereitung Verdickungsmittel in einer Gesamtkonzentration von 0,1 bis 4 Gewichts-% und bevorzugt von 0,3 bis 1,5 Gewichts-%, jeweils bezogen auf das Gesamtgewicht der Zubereitung enthält.

Es ist erfindungsgemäß vorteilhaft, wenn das Gewichtsverhältnis von Ethanol zu UV-A-Lichtschutzfilter (Gesamtmenge) in der Zubereitung von 50:1 bis 1:1 und bevorzugt von 40:1 bis 10:1 betragt

Ebenso ist es erfindungsgemäß vorteilhaft, wenn das Gewichtsverhältnis von bei 20 °C flüssigen Ölkomponenten (Gesamtmenge) zu UV-A-Lichtschutzfilter Gesamtmenge) in der Zubereitung von 25:1 bis 1:1 und bevorzugt von 15:1 bis 4:1 betragt

Die erfindungsgemäße Zubereitung kann erfindungsgemäß vorteilhaft Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmo-nomethyl, -monoethyl- oder -monobutylether, Diethylenglykolmonomethyl- oder - monoethylether und analoge Produkte, Polymere, Schaumstabilisatoren, Elektrolyte, Selbstbrauner, Repellentien enthalten. Soll die erfindungsgemäße Zubereitung Selbstbräuner enthalten, so ist der Einsatz von Dihydroxyaceton in einer Konzentration von bis Gewichts-%, bezogen auf das Gesamtgewicht der Zubereitung, erfindungsgemäß bevorzugt.

Erfindungsgemäß vorteilhaft enthalten die erfindungsgemäßen Zubereitungen einen oder mehrere Filmbildner. Filmbildner im Sinne der vorliegenden Erfindung sind Stoffe unterschiedlicher Zusammensetzung, die durch die folgende Eigenschaft charaktensiert sind: Löst man einen Filmbildner in Wasser oder anderen geeigneten Lösungsmitteln und trägt die Losung dann auf die Haut auf, so bildet er nach dem Verdunsten des Losemittels einen Film aus, der im wesentlichen eine Schutzfunktion hat.

Es ist insbesondere von Vorteil, die Filmbildner aus der Gruppe der Polymere auf Basis von Polyvinylpyrrolidon (PVP) 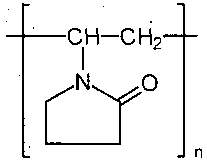 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhaltlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp erhaltlich ist, und/oder Polyisobuten, erhaltlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als gunstige, aber dennoch fakultativ zu verwendende Antioxidantien konnen alle für kosmetische Anwendungen geeigneten oder gebräuchlichen Antioxidantien verwendet werden.

Besonders vorteilhaft im Sinne der vorliegenden Erfindung konnen wasserlösliche Antioxidantien eingesetzt werden, wie beispielsweise Vitamine, z. B. Ascorbinsaure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wahlen.

Es ist insbesondere vorteilhaft, wenn die kosmetischen Zubereitungen gemaß der vorliegenden Erfindung kosmetische Wirkstoffe enthalten, wobei bevorzugte Wirkstoffe Antioxidantien sind, welche die Haut vor oxidativer Beanspruchung schutzen konnen

Weitere vorteilhafte Wirkstoffe im Sinne der vorliegenden Erfindung sind natürliche Wirkstoffe und/oder deren Derivate, wie z. B. alpha-Liponsäure, Folsaure, Phytoen, D-Biotin, Coenzym Q10, alpha-Glucosylrutin, Carnitin, Carnosin, natürliche und/oder synthetische Isoflavonoide, Kreatin, Kreatinin, Taunn und/oder ß-Alanin sowie 8-Hexadecen-1,16-dicarbonsaure (Dioic acid, CAS-Nummer 20701-68-2; vorlaufige INCI-Bezeichnung Octadecendioic acid) und/oder Licochalcon A

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfaltchen, Juckreiz, verminderte Rückfettung (z. B. nach dem Waschen), sichtbare Gefaßerweiterungen (Teleangiektasien, Cuperosis), Schlaffheit und Ausbildung von Falten und Faltchen, lokale Hyper-, Hypo- und Fehlpigmentierungen (z. B Altersflecken), vergroßerte Anfälligkeit gegenüber mechanischem Stress (z. B. Rissigkeit und dergleichen) und ermüdete Haut Weiterhin vorteilhaft eignen sie sich gegen das Erscheinungsbild der trockenen bzw. rauhen Haut.

Die kosmetischen Zubereitungen gemaß der Erfindung können kosmetische Hilfsstoffe enthalten, wie sie üblicherweise in solchen Zubereitungen verwendet werden, z. B Konservierungsmittel, Konservierungshelfer, Komplexbildner, Bakterizide, Parfume, Substanzen zum Verhindern oder Steigern des Schäumens, Farbstoffe, Pigmente, die eine färbende Wirkung haben, anfeuchtende und/oder feuchhaltende Substanzen, Füllstoffe, die das Hautgefuhl verbessern oder andere übliche Bestandteile einer kosmetischen oder dermatologischen Formulierung.

Vorteilhaft im Sinne der vorliegenden Erfindung sind Zubereitungen zur Pflege der Haut: sie konnen dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsachlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Die erfindungsgemäßen Zubereitungen können zum Schutz vor Hautalterung (insbesondere zum Schutz vor UV-bedingter Hautalterung) sowie als Sonnenschutzmittel verwendet werden.

Die erfindungsgemäße Zubereitung weist vorteilhaft einen pH-Wert von 5 bis 8 auf. Dieser kann durch die herkömmlichen Säuren, Basen und Puffersysteme eingestellt werden.

Ferner ist es im Sinne der vorliegenden Erfindung, der erfindungsgemäßen Zubereitung Perlglanzpigmente, Glimmer, Glitterstoffe, Kügelchen und/oder Effektpigmente, Peelingpartikel, wie z.B. Polyethylen, zuzusetzen, um die Zubereitung optisch attraktiver zu gestalten und/oder einen Zusatznutzen herbeizufuhren. Auch kann das erfindungsgemäße Reinigungsgel mit Gasblasen, insbesondere Luftblasen, oder Schlieren, insbesondere Farbschlieren, in für die Erfindung vorteilhafter Weise versehen sein.

Ein für die erfindugsgemäße Zubereitung vorteilhaftes Verpackungsbehältnis wird vorteilhaft aus Glas oder Polyethylenterephthalat (PET) oder Highdensity-PET (HDPE) gebildet.

Es ist vorteilhaft, die erfindungsgemäße Zubereitung als Spray oder als Gel anzuwenden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele für Gele

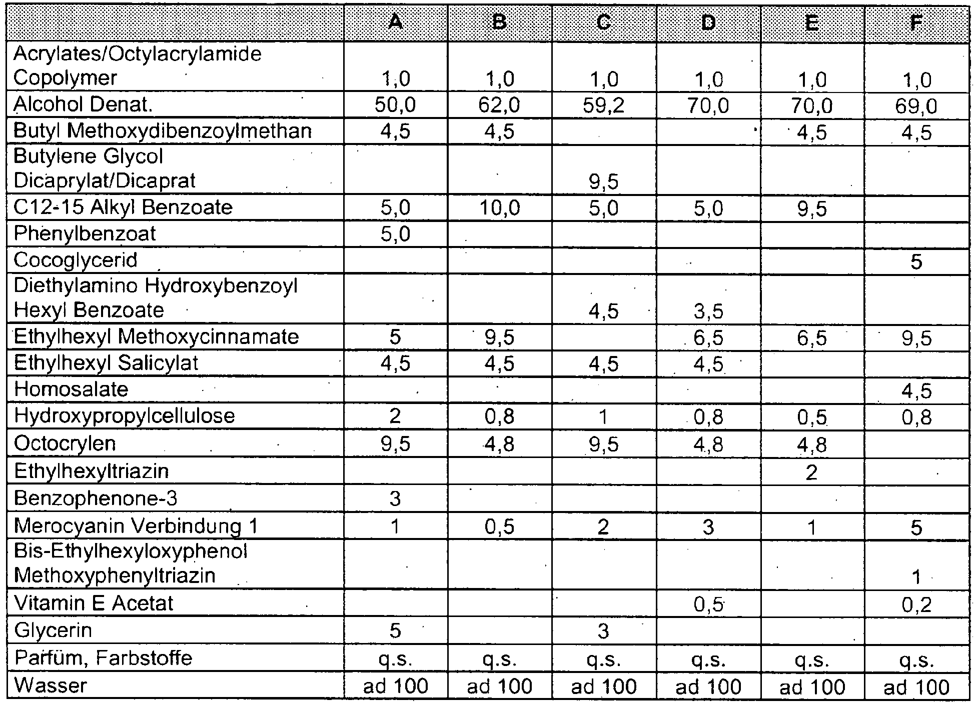

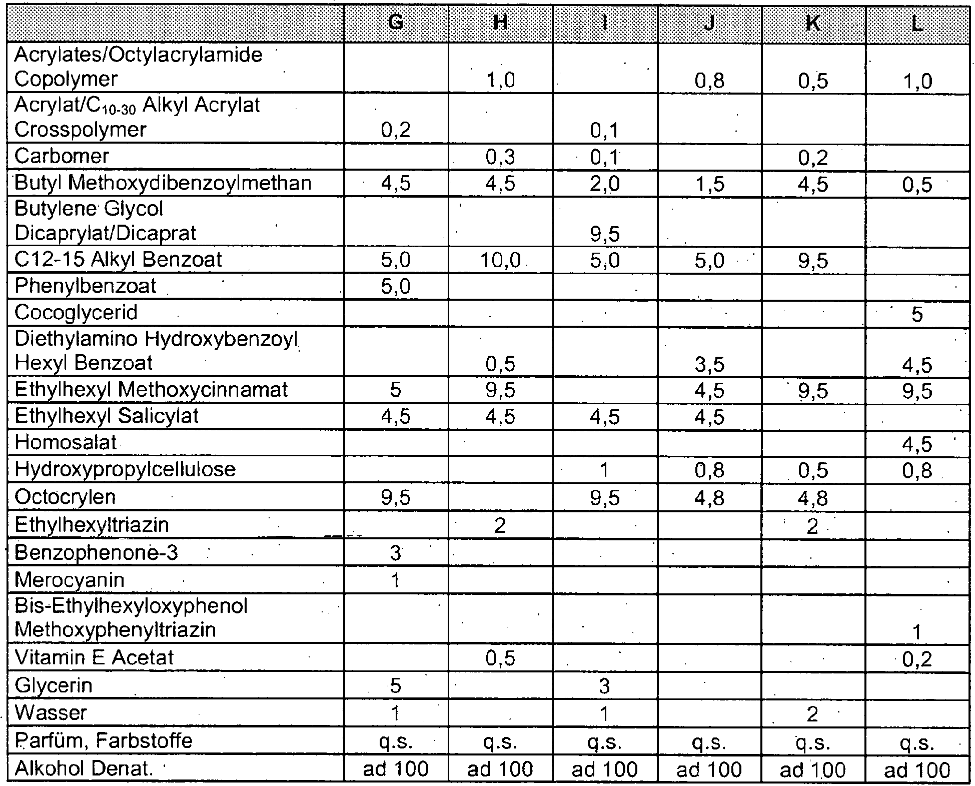

### Beispiele für Sprays

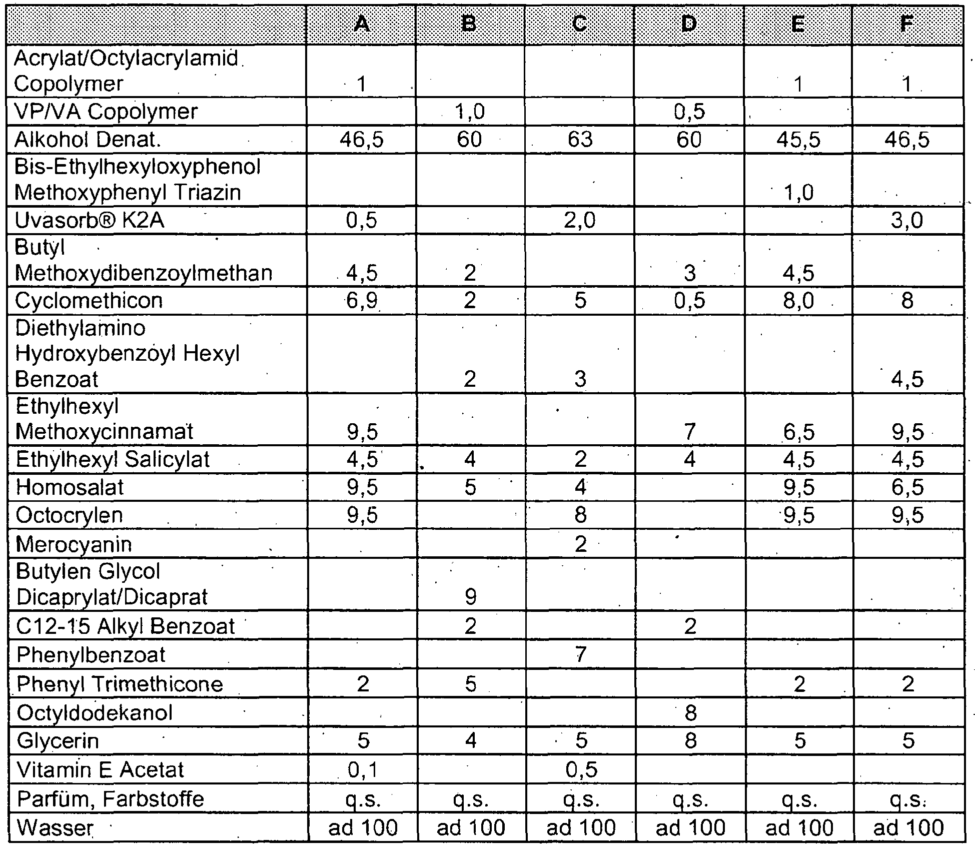

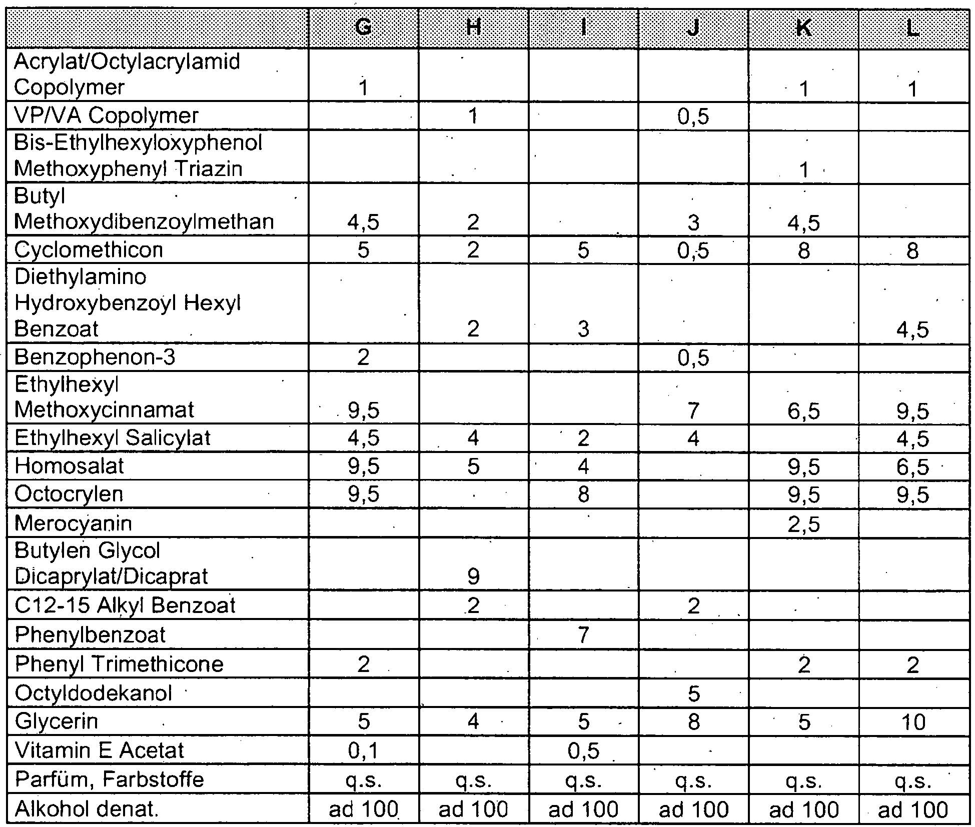

## Patentansprüche

1. Transparentes kosmetisches Sonnenschutzmittel enthaltend
a) von 20 bis 80 Gewichts-% Ethanol,
b) von 15 bis 45 Gewichts-% bei 20 °C flüssige Ölkomponenten,
c) von 1,0 bis 12 Gewichts-% UV-A-Lichtschutzfilter aus der Gruppe der Verbindungen 2-(4'-Diethylamino-2'-hydoxybenzoyl)-benzoesäurehexylester und 4-(tert.-Butyl)-4'-methoxydibenzoylmethan,
wobei sich die Konzentrationsangaben auf das Gesamtgewicht der Zubereitung beziehen,
**dadurch gekennzeichnet, dass** die bei 20°C flüssigen Ölkomponenten gewählt werden aus der Gruppe der Verbindungen C₁₂₋₁₅-Alkylbenzoat, Octyldodekanol, Phenethylbenzoat, Cocoglycerid, 2-Ethylhexyl-2-cyano-3,3-diphenylacrylat; Homomenthylsalicylat, Ethylhexylsalicylat, 4-Methoxyzimtsäureisoamylester, Dibutyladipat, 4-Methoxyzimtsäure(2-ethylhexyl)ester, Cyclomethicon, Dimethicon, sowie **dadurch gekennzeichnet, dass** der Wassergehalt der Zubereitung höchstens 4,5 Gewichts-%, bezogen auf das Gesamtgewicht der Zubereitung, beträgt.

2. Sonnenschutzmittel nach Anspruch 1, **dadurch gekennzeichnet, dass** als UV-A-Lichtschutzfilter 4-(tert.-Butyl)-4'-methoxydibenzoylmethan eingesetzt wird.

3. Sonnenschutzmittel nach einem der vorhergehenden Ansprüche, **dadurch gekennzeichnet, dass** die Zubereitung einen oder mehrere UV-Lichtschutzfilter gewählt aus der Gruppe der Verbindungen Dioctylbutylamidotriazon; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Terephthalidendicamphersulfonsäure; 4-(Dimethylamino)benzoesäure-amylester; 4-Methoxybenzalmalon-säuredi(2-ethylhexyl)ester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI:Bis-Ethylhexyloxyphenol Methoxyphenyl Triazin); Dioctylbutylamidotriazon (INCI: Diethylhexyl-Butamidotriazone); 4,4',4"-(1,3,5-Triazin-2,4,6-triyltriimino)-tris-benzoë-säure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine, 2,4,6-Tribiphenyl-4-yl-1,3,5-triazin; Titandioxid; Zinkoxid in einer Konzentration von 0,01 bis 40 Gewichts-%, bezogen auf das Gesamtgewicht der Zubereitung, enthält.

4. Sonnenschutzmittel nach einem der vorhergehenden Ansprüche, **dadurch gekennzeichnet, dass** das Gewichtsverhältnis von Ethanol zu UV-A-Lichtschutzfilter (Gesamtmenge) von 50:1 bis 1:1 beträgt.

5. Sonnenschutzmittel nach einem der vorhergehenden Ansprüche, **dadurch gekennzeichnet, dass** das Gewichtsverhältnis von bei 20 °C flüssigen Ölkomponenten (Gesamtmenge) zu UV-A-Lichtschutzfilter Gesamtmenge) von 25:1 bis 1:1 beträgt.

6. Sonnenschutzmittel nach einem der vorhergehenden Ansprüche, **dadurch gekennzeichnet, dass** das Sonnenschutzmittel von 50 bis 85 Gewichts-% Ethanol, bezogen auf das Gesamtgewicht der Zubereitung, enthält.

7. Sonnenschutzmittel nach einem der vorhergehenden Ansprüche, **dadurch gekennzeichnet, dass** das Sonnenschutzmittel von 60 bis 80 Gewichts-% Ethanol, bezogen auf das Gesamtgewicht der Zubereitung, enthält.

## Claims

1. Transparent cosmetic sunscreen composition comprising
a) from 20 to 80% by weight of ethanol,
b) from 15 to 45% by weight of oil components that are liquid at 20°C,
c) from 1.0 to 12% by weight of UV-A light protection filters form the group of the compounds hexyl 2-(4'-diethylamino-2'-hydroxy-benzoyl)benzoate and 4-(tert-butyl)-4'-methoxy-dibenzoylmethane,
where the concentration data refer to the total weight of the preparation,
**characterized in that** the oil components that are liquid at 20°C are selected from the group of the compounds C₁₂₋₁₅-alkyl benzoate, octyldodecanol, phenethyl benzoate, cocoglyceride, 2-ethylhexyl 2-cyano-3,3-diphenylacrylate; homomenthyl salicylate, ethylhexyl salicylate, isoamyl 4-methoxycinnamate, dibutyl adipate, 2-ethylhexyl 4-methoxycinnamate, cyclomethicone, dimethicone, and also **characterized in that** the water content of the preparation is at most 4.5% by weight, based on the total weight of the preparation.

2. Sunscreen composition according to Claim 1, **characterized in that** 4-(tert-butyl)-4'-methoxy-dibenzoylmethane is used as UV-A light protection filter.

3. Sunscreen composition according to one of the preceding claims, **characterized in that** the preparation comprises one or more UV light protection filters selected from the group of the compounds dioctylbutylamidotriazone; phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidene-methyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]-disiloxanyl]propyl]phenol; 3-(4-methylbenzylidene)camphor; 3-benzylidenecamphor; terephthalidenedicamphorsulphonic acid; amyl 4-(dimethylamino)benzoate; di(2-ethylhexyl) 4-methoxybenzalmalonate; 2-hydroxy-4-methoxybenzophenone; 2-hydroxy-4-methoxy-4'-methylbenzo-phenone; 2,2'-dihydroxy-4-methoxybenzophenone; 2-ethylhexyl 2-hydroxybenzoate; dimethicodiethyl-benzalmalenate; 3-(4-(2,2-bis-ethoxycarbonyl-vinyl)phenoxy)propenyl)methoxysiloxane/dimethyl-siloxane copolymer; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); tris(2-ethylhexyl) 4,4',4"-(1,3,5-triazine-2,4,6-triyltriimino)tris-benzoate (also: 2,4,6-tris[anilino(p-carbo-2'-ethyl-1'-hexyloxyl)]-1,3,5-triazine (INCI: Ethyl-hexyl Triazone); merocyanines, 2,4,6-tribiphenyl-4-yl-1,3,5-triazine; titanium dioxide, zinc oxide in a concentration of from 0.01 to 40% by weight, based on the total weight of the preparation.

4. Sunscreen composition according to one of the preceding claims, **characterized in that** the weight ratio of ethanol to UV-A light protection filter (total amount) is from 50:1 to 1:1.

5. Sunscreen composition according to one of the preceding claims, **characterized in that** the weight ratio of oil components that are liquid at 20°C (total amount) to UV-A light protection filter (total amount) is from 25:1 to 1:1.

6. Sunscreen composition according to one of the preceding claims, **characterized in that** the sunscreen composition comprises from 50 to 85% by weight of ethanol, based on the total weight of the preparation.

7. Sunscreen composition according to one of the preceding claims, **characterized in that** the sunscreen composition comprises from 60 to 80% by weight of ethanol, based on the total weight of the preparation.

## Revendications

1. Produit antisolaire cosmétique transparent, contenant
a) de 20 à 80 % en poids d'éthanol,
b) de 15 à 45 % en poids de composants huileux liquides à 20 °C,
c) de 1,0 à 12 % en poids de filtre(s) photoprotecteur(s) anti-UV-A choisi(s) dans le groupe des composés 2-(4'-diéthylamino-2'-hydroxybenzoyl)-benzoate d'hexyle et 4-(tert-butyl)-4'-méthoxydibenzoylméthane,
les données de concentrations se rapportant au poids total de la préparation,
**caractérisé en ce que** les composants huileux liquides à 20°C sont choisis dans le groupe des composés benzoate d'alkyle en C₁₂-C₁₅, octyldodécanol, benzoate de phénéthyle, glycéride de coco, 2-cyano-3,3-diphényl-acrylate de 2-éthylhexyle, salicylate d'homomenthyle, salicylate d'éthylhexyle, 4-méthoxycinnamate d'iso-amyle, adipate de dibutyle, 9-méthoxycinnamate de 2-éthylhexyle, cyclométhicone, diméthicone, ainsi que **caractérisé en ce que** la teneur en eau de la préparation est au maximum de 4,5 % en poids, par rapport au poids total de la préparation.

2. Produit antisolaire selon la revendication 1, **caractérisé en ce qu'**on utilise comme filtre photoprotecteur anti-UV-A le 4-(tert-butyl)-4'-méthoxydibenzoylméthane.

3. Produit antisolaire selon l'une quelconque des revendications précédentes, **caractérisé en ce que** la préparation contient un ou plusieurs filtres photoprotecteurs anti-UV choisi(s) dans le groupe des composés dioctylbutylamidotriazone ; sels d'acide phénylène-1,4-bis-(2-benzimidazyl)-3,3'-5,5'-tétra-sulfonique ; sels d'acide 2-phénylbenzimidazole-5-sulfonique ; 1,4-di(2-oxo-10-sulfo-3-bornylidène-méthyl)-benzène et ses sels ; sels d'acide 4-(2-oxo-3-bornylidèneméthyl)benzènesulfonique ; sels d'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; acide téréphtalidènedicamphosulfonique ; 4-(diméthyl-amino)benzoate d'amyle ; 4-méthoxybenzalmalonate de di(2-éthylhexyle) ; 2-hydroxy-4-méthoxybenzophénone, 2-hydroxy-4-méthoxy-4'-méthylbenzophénone ; 2,2'-dihydroxy-4-méthoxybenzophénone ; 2-hydroxybenzoate de 2-éthylhexyle ; benzalmalonate de diméthicodiéthyle ; copolymère 3-(4-(2,2-bis-éthoxycarbonylvinyl)-phénoxy)-propényl)-méthoxysiloxane / diméthylsiloxane ; 2,4-bis-{[4-(2-éthyl-hexyloxy)-2-hydroxy]-phényl}-6-(4-méthoxy-phényl)-1,3,5-triazine (INCI : bis-ethylhexyloxyphenol methoxyphenyl triazin) ; dioctylbutylamidotriazone (INCI : diethylhexyl-butamidotriazone) ; 4,4',4"-(1.3,5-triazin-2,4,6-triyltriimino)-tris-benzoate de tris(2-éthylhexyle) (également : 2,4,6-tris-[anilino-(p-carbo-2'-éthyl-1'-hexyloxy)]-1,3,5-triazine (INCI : ethylhexyl triazone) ; mérocyanines, 2,4,6-tribiphényl-4-yl-1,3,5-triazine ; dioxyde de titane ; oxyde de zinc à une concentration de 0,01 à 40 % en poids, par rapport au poids total de la préparation.

4. Produit antisolaire selon l'une quelconque des revendications précédentes, **caractérisé en ce que** le rapport pondéral de l'éthanol aux filtres photoprotecteurs anti-UV-A (quantité totale) vaut de 50:1 à 1:1.

5. Produit antisolaire selon l'une quelconque des revendications précédentes, **caractérisé en ce que** le rapport pondéral des composants huileux liquides à 20 °C (quantité totale) aux filtres photoprotecteurs anti-UV-A (quantité totale) vaut de 25:1 à 1:1.

6. Produit antisolaire selon l'une quelconque des revendications précédentes, **caractérisé en ce que** le produit antisolaire contient de 50 à 85 % en poids d'éthanol, par rapport au poids total de la préparation.

7. Produit antisolaire selon l'une quelconque des revendications précédentes, **caractérisé en ce que** le produit antisolaire contient de 60 à 80 % en poids d'éthanol, par rapport au poids total de la préparation.
